# EUROPÄISCHE PATENTANMELDUNG

(11) **EP 3 557 589 A1**
(43) Veröffentlichungstag der Anmeldung: **23.10.2019**
(21) Anmeldenummer: 18168173.5
(22) Anmeldetag: 19.04.2018
(51) Int. Cl.: G16H 40/40

(54) **VERFAHREN UND SYSTEM ZUR VORHERSAGE VON SYSTEMAUSFÄLLEN BEI EINEM MEDIZINISCHEN SYSTEM**

(71) Anmelder: Siemens Aktiengesellschaft, 80333 München (DE)
(72) Erfinder: HIPP, Tobias, 90482 Nürnberg (DE); MECKING, Marie, 91052 Erlangen (DE); KROMPASS, Denis, 81549 München (DE)

(57) **Zusammenfassung**

Vorrichtung und Verfahren zur Vorhersage von Systemausfällen bei einem medizinischen System (2), wobei die Vorrichtung (1) aufweist: eine Erfassungseinheit (3) zur Erfassung von Sensordaten (SD) und/oder Ereignisdaten (ED) hinsichtlich mindestens einer Systemkomponente des medizinischen Systems (2); eine Berechnungseinheit (4) mit mindestens einem darin implementierten künstlichen neuronalen Netzwerk (5) zur Berechnung einer Ausfallwahrscheinlichkeit (P) für einen zukünftigen Ausfall der Systemkomponente des medizinischen Systems (2), und eine Ausgabeeinheit (6) zur Ausgabe der berechneten Ausfallwahrscheinlichkeit (P) der Systemkomponente des medizinischen Systems (2).

## Beschreibung

Medizinische Geräte bzw. medizinische Systeme unterliegen wie alle technischen Vorrichtungen einem Verschleiß und sind daher in Stand zu halten. Die Instandhaltung dient der Verringerung der Verschleißgeschwindigkeit sowie der Vermeidung von Fehlfunktionen. Die Instandhaltung kann allgemein korrektiv oder präventiv erfolgen. Die korrektive Instandhaltung, d.h. die außerhalb bedingte bzw. schadensabhängige Instandhaltung wird erst nach einem Ausfall einer Systemkomponente des medizinischen Systems vorgenommen. Eine weitere Möglichkeit bietet die präventive (vorbeugende bzw. vorausbestimmte) Instandhaltung (preventive maintenance). Hierbei erfolgt die Instandsetzung bzw. Wartung des medizinischen Systems in festgelegten Zeitintervallen, um Ausfälle von Systemkomponenten des medizinischen Systems bzw. des gesamten medizinischen Systems weitgehend zu vermeiden. Bei der prädiktiven bzw. zustandsorientierten Instandhaltungsstrategie (predictive maintenance) wird das betrachtete medizinische System durch kontinuierliche oder diskontinuierliche Inspektion überwacht.

Insbesondere bei medizinischen Systemen ist es notwendig Betriebsausfallzeiten zu vermeiden. Hierzu ist es notwendig Systemausfälle eines medizinischen Systems rechtzeitig zu erkennen, um entsprechende Wartungs- bzw. Reparaturmaßnahmen noch vor einem Systemausfall vornehmen zu können.

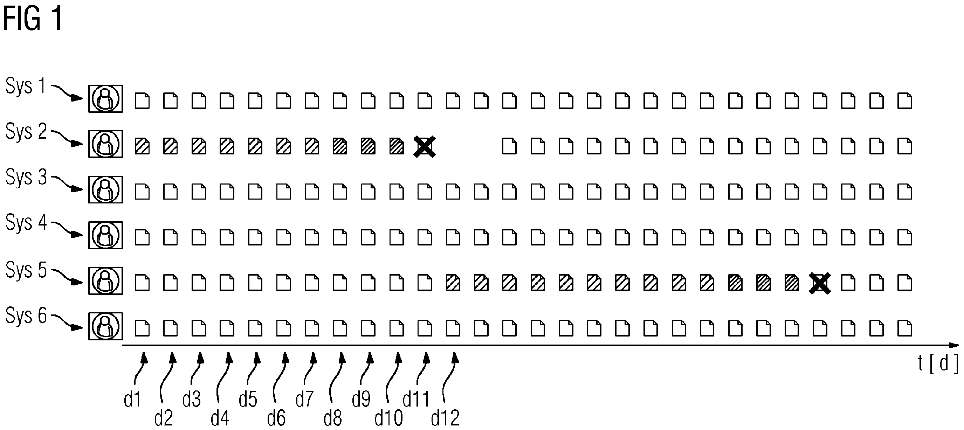

Fig. 1 zeigt schematisch, welche Anforderungen an die prädiktive Instandhaltung von medizinischen Systemen bestehen. Es werden in Fig. 1 sechs verschiedene medizinische Systeme bzw. Geräte über die Zeit betrachtet. Jedes überwachte medizinische System überträgt täglich Log-Daten bzw. Ereignisdaten. Bei dem in Fig. 1 dargestellten Schema wird mit einem Kreuz angedeutet, dass das betrachtete medizinische System ausfällt. Beispielsweise fällt das zweite System Sys2 in dem betrachteten Beispiel am 11. Tag (d11) aus. Wird der Alarm bzw. das Alarmsignal lediglich in einem kurzen Zeitbereich vor dem Systemausfall, beispielsweise innerhalb eines kritischen Zeitraums von drei Tagen (d8, d9, d10) generiert, kommt dieser Alarm bzw. dieses Warnsignal zu spät, um einen Systemausfall des Systems noch zu vermeiden. Dies liegt beispielsweise daran, dass notwendige Ersatzteile nicht vorhanden sind bzw. beim Hersteller angefordert werden müssen. Weiterhin ist es möglich, dass Servicetechniker in diesem Zeitraum nicht verfügbar sind. Wird das Alarmsignal vorher generiert, d.h. beispielsweise in einem Zeitraum, der vor dem kritischen Zeitraum liegt, können die notwendigen Reparatur- bzw. Wartungsmaßnahmen noch rechtzeitig eingeleitet werden, um einen Systemausfall des medizinischen Gerätes zu vermeiden.

Es ist daher eine Aufgabe der vorliegenden Erfindung ein Verfahren und ein System zur rechtzeitigen Vorhersage von Systemausfällen bei einem medizinischen System zu schaffen, sodass Wartungs- bzw. Instandhaltungsmaßnahmen rechtzeitig vorgenommen werden, um einen Systemausfall des medizinischen Systems zu verhindern.

Diese Aufgabe wird erfindungsgemäß durch ein Verfahren zur Vorhersage von Systemausfällen bei einem medizinischen System mit den in Patentanspruch 1 angegebenen Merkmalen gelöst.

Die Erfindung schafft demnach ein Verfahren zur Vorhersage von Systemausfällen bei einem medizinischen System mit den Schritten:
Erfassen von Sensordaten und/oder Ereignisdaten hinsichtlich mindestens einer Systemkomponente des medizinischen Systems, Auswerten der erfassten Sensordaten und/oder der erfassten Ereignisdaten durch mindestens ein trainiertes künstliches neuronales Netzwerk zur Berechnung einer Ausfallwahrscheinlichkeit eines zukünftigen Ausfalls der Systemkomponente des medizinischen Systems, und
Ausgeben der berechneten Ausfallwahrscheinlichkeit der Systemkomponente des medizinischen Systems.

Bei einer möglichen Ausführungsform des erfindungsgemäßen Verfahrens weist das künstliche neuronale Netzwerk ein mehrschichtiges rekurrentes neuronales Netzwerk auf.

Bei einer weiteren möglichen Ausführungsform des erfindungsgemäßen Verfahrens werden berechnete Ausfallwahrscheinlichkeiten an eine Auswerteeinheit ausgegeben werden, welche die Ausfallwahrscheinlichkeiten auf Basis von vordefinierten Auswerteregeln zur Bestimmung von Maßnahmen, welche einen teilweisen oder vollständigen Systemausfall des medizinischen Systems verhindern, auswertet.

Bei einer weiteren möglichen Ausführungsform des erfindungsgemäßen Verfahrens generiert die Auswerteeinheit ein Systemausfallwarnsignal, sobald die berechneten Ausfallwahrscheinlichkeiten mehrfach hintereinander ansteigen und/oder einen vorgegebenen Schwellenwert überschreiten.

Bei einer weiteren möglichen Ausführungsform des erfindungsgemäßen Verfahrens wird das generierte Systemausfallwarnsignal an ein entferntes Service-Center übertragen und/oder über eine Nutzerschnittstelle des medizinischen Systems ausgegeben.

Bei einer weiteren möglichen Ausführungsform des erfindungsgemäßen Verfahrens werden die erfassten Zeitreihen der Sensordaten und/oder der Ereignisdaten zur Ermittlung von Zeitreihen gefiltert, die kurzfristige und langfristige zeitliche Trends innerhalb der erfassten Zeitreihen der Sensordaten und/oder der Ereignisdaten wiedergeben.

Bei einer weiteren möglichen Ausführungsform des erfindungsgemäßen Verfahrens werden die gefilterten Zeitreihen der Sensordaten und/oder der Ereignisdaten verdeckten Schichten verschiedener künstlicher neuronaler Netzwerke zugeführt, die eine gemeinsame Ausgabeschicht zum Berechnen der Ausfallwahrscheinlichkeit der Systemkomponente des Systems besitzen.

Bei einer möglichen Ausführungsform des erfindungsgemäßen Verfahrens werden Sensordaten und/oder Ereignisdaten hinsichtlich eines Filaments eines Röntgensystems erfasst.

Die Erfindung schafft ferner ein medizinisches System mit den in Anspruch 9 angegebenen Merkmalen.

Die Erfindung schafft demnach ein medizinisches System mit einer Erfassungseinheit zur Erfassung von Sensordaten und/oder Ereignisdaten hinsichtlich mindestens einer Systemkomponente des medizinischen Systems,
einer Berechnungseinheit mit mindestens einem darin implementierten künstlichen neuronalen Netzwerk zur Berechnung einer Ausfallwahrscheinlichkeit für einen zukünftigen Ausfall der Systemkomponente des medizinischen Systems und
einer Ausgabeeinheit zur Ausgabe der berechneten Ausfallwahrscheinlichkeit der Systemkomponente des medizinischen Systems.

Bei einer möglichen Ausführungsform des erfindungsgemäßen medizinischen Systems weist das künstliche neuronale Netzwerk ein mehrschichtiges rekurrentes neuronales Netzwerk auf, das mehrere verdeckte Schichten und eine Ausgabeschicht enthält.

Bei einer möglichen Ausführungsform des erfindungsgemäßen medizinischen Systems weisen die verdeckten Schichten des in der Berechnungseinheit implementierten rekurrenten neuronalen Netzwerkes jeweils LSTM-Module auf.

Bei einer weiteren möglichen Ausführungsform des erfindungsgemäßen medizinischen Systems weisen die verdeckten Schichten des in der Berechnungseinheit implementierten rekurrenten neuronalen Netzwerkes GRU-Module auf.

Bei einer möglichen Ausführungsform des erfindungsgemäßen medizinischen Systems bildet die erste verdeckte Schicht des rekurrenten neuronalen Netzwerkes eine Eingabeschicht, welche über eine Schnittstelle Sensordaten und/oder Ereignisdaten von einem Zwischenspeicher der Erfassungseinheit des medizinischen Systems erhält.

Bei einer weiteren möglichen Ausführungsform des erfindungsgemäßen medizinischen Systems sind Filtereinheiten vorgesehen, welche Zeitreihen der durch die Erfassungseinheit erfassten Sensordaten und/oder Zeitreihen der durch die Erfassungseinheit erfassten Ereignisdaten zur Ermittlung gefilterter Zeitreihen filtern, welche kurzfristige und langfristige zeitliche Trends innerhalb der erfassten Sensordaten und/oder Ereignisdaten wiedergeben, wobei die durch die Filtereinheiten gefilterten Zeitreihen Eingabeschichten verschiedener innerhalb der Berechnungseinheit implementierter rekurrenter neuronaler Netzwerke zugeführt werden.

Bei einer weiteren möglichen Ausführungsform des erfindungsgemäßen medizinischen Systems gibt die Ausgabeeinheit des medizinischen Systems die durch die Berechnungseinheit berechneten Ausfallwahrscheinlichkeiten der Systemkomponente an eine Auswerteeinheit des medizinischen Systems aus, welche die Ausfallwahrscheinlichkeiten auf Basis vordefinierter Auswerteregeln zur Generierung eines Systemausfallwarnsignales auswertet, das einen teilweisen oder vollständigen Systemausfall des medizinischen Systems anzeigt.

Bei einer weiteren möglichen Ausführungsform des erfindungsgemäßen medizinischen Systems weist die Auswerteeinheit eine Datenschnittstelle zur Ausgabe des generierten Systemausfallwarnsignales an ein Service-Center und/oder eine Nutzerschnittstelle zur Ausgabe des generierten Systemausfallwarnsignales an einen Nutzer des medizinischen Systems auf.

Im Weiteren werden mögliche Ausführungsformen des erfindungsgemäßen Verfahrens und des erfindungsgemäßen Systems unter Bezugnahme auf die beigefügten Figuren näher erläutert.

Es zeigen:
- Fig. 1: eine schematische Darstellung zur Erläuterung von Anforderungen, die an eine prädiktive Instandhaltung eines medizinischen Systems gestellt werden;
- Fig. 2: ein Blockschaltbild zur Darstellung eines Ausführungsbeispiels eines erfindungsgemäßen medizinischen Systems;
- Fig. 3, 4: schematische Darstellungen möglicher exemplarischer Ausführungsbeispiele eines erfindungsgemäßen medizinischen Systems;
- Fig. 5: ein Ablaufdiagramm zur Darstellung eines möglichen Ausführungsbeispiels eines erfindungsgemäßen Verfahrens zur Vorhersage von Systemausfällen;
- Fig. 6: ein Diagramm zur Erläuterung der Generierung eines Systemausfallwarnsignals bei einer möglichen Ausführungsform eines erfindungsgemäßen Verfahrens zur Vorhersage von Systemausfällen.

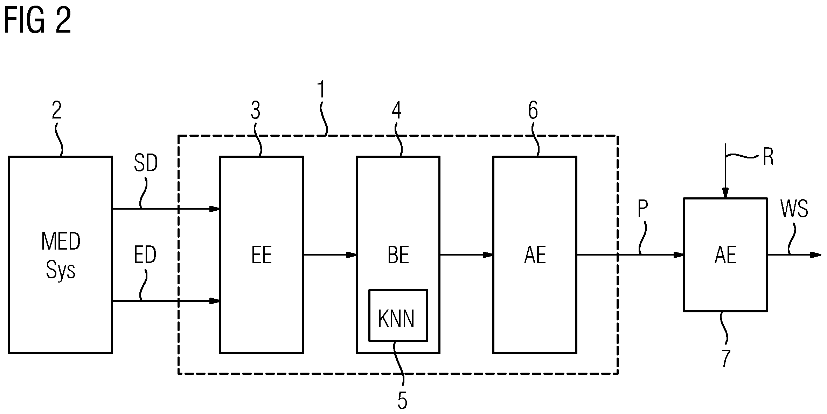

Fig. 2 zeigt ein Blockschaltbild zur Darstellung eines möglichen Ausführungsbeispiels eines erfindungsgemäßen Systemausfallvorhersagesystems. Bei dem in Fig. 2 dargestellten Ausführungsbeispiel ist das Vorhersagesystem 1 mit einem überwachenden medizinischen System 2 mittels einer oder mehreren Datenschnittstellen gekoppelt. Das überwachte medizinische System bzw. medizinische Gerät 2 kann mehrere integrierte Sensoren enthalten, welche Sensordaten liefern. Die Sensordaten werden durch Zeitreihen gebildet, welche in einem Rohdatenformat, in aggregierter Form oder in einer Mischform, weiterverarbeitet werden können. Weiterhin werden durch das medizinische System 2 Log-Files generiert, welche Log-File-Einträge mit Ereignisdaten umfassen. Log-Files sind allgemein Dateien, in denen Prozessdaten einer Betrachtungseinheit bzw. einer oder mehrerer Systemkomponenten gespeichert werden. Diese Ereignisdaten umfassen Warn- und Fehler-Events hinsichtlich Systemkomponenten des medizinischen Systems 2. Daneben werden für die einzelnen Systemkomponenten Leerlaufnachrichten, Startmeldungen und Stoppmeldungen, Prozessschritte, Sensorwerte, Fehlermeldungen und Warnungen aufgezeichnet und in Log-Files gespeichert. Bei dem überwachten medizinischen System bzw. medizinischen Gerät 2 kann es sich beispielsweise um eine Röntgenröhre handeln. Bei einer Röntgenröhre treten durch starke Erhitzungen an einer Glühkathode Elektronen aus dieser Kathode aus und sammeln sich als Elektronenwolke um ein Filament. Die Elektronen werden durch die Hochspannung zu einer Anode beschleunigt und dort stark abgebremst, wodurch eine Bremsstrahlung und eine charakteristische Röntgenstrahlung entstehen. Der Defekt einer jeden Systemkomponente der Röntgenröhre kann zu einem vollständigen oder partiellen Ausfall der Röntgenröhre führen. Die Filamente werden über den Nutzungszeitraum der Röntgenröhre kontinuierlich dünner, da die Erhitzung der Glühkathode zu einer Materialerosion des Filaments führt. Ist das Filament der Röntgenröhre zu dünn, kann es brechen oder sogar einen Kurzschluss verursachen, wodurch es unbrauchbar wird. Das Systemausfallvorhersagesystem 1 kann eine oder mehrere Systemkomponenten des medizinischen Gerätes 2 überwachen.

Bei dem in Fig. 2 dargestellten Ausführungsbeispiel weist die Vorrichtung 1 zur Vorhersage von Systemausfällen bei dem überwachten medizinischen System 2 eine Erfassungseinheit 3 zur Erfassung von Sensordaten und/oder Ereignisdaten hinsichtlich mindestens einer Systemkomponente des medizinischen Systems 2 auf. Ferner umfasst die Vorrichtung 1 eine Berechnungseinheit 4 mit mindestens einem darin implementierten künstlichen neuronalen Netzwerk 5 zur Berechnung einer Ausfallwahrscheinlichkeit für einen zukünftigen Ausfall einer Systemkomponente des medizinischen Systems 2. Ferner weist die Vorrichtung 1 eine Ausgabeeinheit 6 zur Ausgabe der berechneten Ausfallwahrscheinlichkeit der Systemkomponente des medizinischen Systems 2 auf. Die Ausgabeeinheit 6 gibt die durch die Berechnungseinheit 4 berechnete Ausfallwahrscheinlichkeiten P der Systemkomponente an eine Auswerteeinheit 7 aus, welche die Ausfallwahrscheinlichkeiten auf Basis vordefinierter Auswerteregeln R zur Generierung eines Systemausfallwarnsignals WS auswertet, das einen teilweisen oder vollständigen Systemausfall des medizinischen Systems 2 anzeigt. Die Auswerteeinheit 7 kann bei einer möglichen Ausführungsform in der Vorrichtung 1 integriert sein. In einer weiteren möglichen Ausführungsform weist die Auswerteeinheit 7 bzw. die Vorrichtung 1 eine Datenschnittstelle zur Ausgabe des generierten Systemausfallwarnsignales WS an ein entferntes Service-Center auf. Weiterhin kann die Auswerteeinheit 7 bzw. die Vorrichtung 1 eine Nutzerschnittstelle zur Ausgabe des generierten Systemausfallwarnsignales WS an einen Nutzer des medizinischen Systems 2 aufweisen.

Bei dem in der Berechnungseinheit 4 implementierten künstlichen neuronalen Netzwerk 5 handelt es sich vorzugsweise um ein mehrschichtiges rekurrentes neuronales Netzwerk RNN, das mehrere verdeckte Schichten (hidden layers) und eine Ausgabeschicht (dense layer) enthält. Die verdeckten Schichten des rekurrenten neuronalen Netzwerkes 5 weisen bei einer möglichen Ausführungsform jeweils LSTM-Module auf. Bei einer alternativen Ausführungsform weisen die verdeckten Schichten des rekurrenten neuronalen Netzwerkes 5 GRU-Module auf.

Bei einer möglichen Ausführungsform bildet die erste verdeckte Schicht des rekurrenten neuronalen Netzwerkes 5 eine Eingabeschicht, die über eine Schnittstelle Sensordaten und/oder Ereignisdaten von einem Zwischenspeicher der Erfassungseinheit 3 enthält.

Bei einer möglichen Ausführungsform enthält die Berechnungseinheit 4 ferner Filtereinheiten, welche Zeitreihen der durch die Erfassungseinheit 3 erfassten Sensordaten und/oder Zeitreihen der Ereignisdaten zur Ermittlung gefilterter Zeitreihen filtern, welche kurzfristige und langfristige zeitliche Trends innerhalb der erfassten Sensordaten und/oder Ereignisdaten wiedergeben. Die durch die Filtereinheiten gefilterten Zeitreihen können Eingabeschichten verschiedener innerhalb der Berechnungseinheit 4 implementierter rekurrenter neuronaler Netzwerke 5 zugeführt werden.

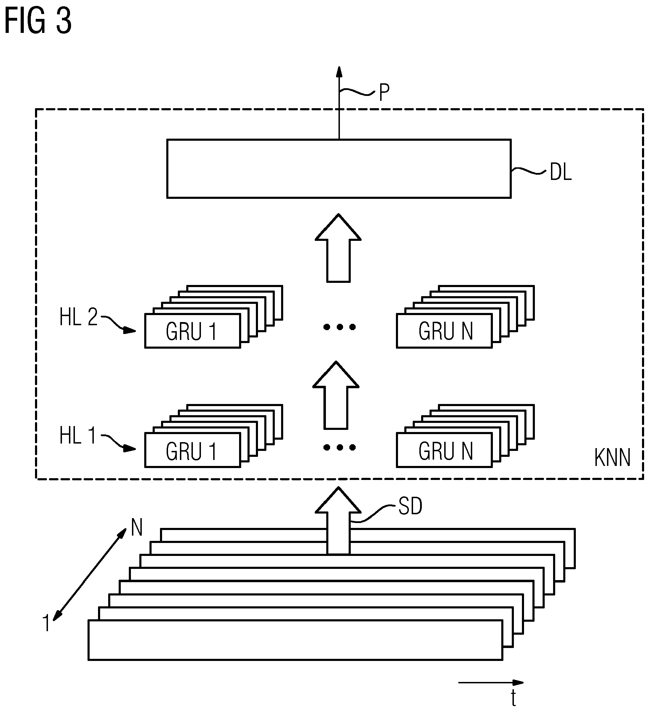

Fig. 3 zeigt ein erstes Ausführungsbeispiel einer in der Vorrichtung 1 enthaltenen Berechnungseinheit 4 mit einem darin implementierten rekurrenten neuronalen Netzwerk RNN. Zeitreihen verschiedener Sensorsignale bzw. Messsignale werden einer Eingabeschicht des rekurrenten neuronalen Netzwerkes zugeführt. In dem dargestellten Ausführungsbeispiel weist das rekurrente neuronale Netzwerk zwei verdeckte Schichten bzw. hidden layers HL auf sowie eine Ausgabeschicht bzw. dense layer DL (fully connected layer). Die Größe der verdeckten Schichten bzw. hidden layers HL beträgt beispielsweise 70 bis 80 Elemente. Die Eingangsdaten umfassen Sequenzen unterschiedlicher Länge beispielsweise 300 Scans mit jeweils 15 Messwerten unterschiedlicher Sensoren.

Die beiden verdeckten Schichten HL₁, HL₂ des rekurrenten neuronalen Netzwerkes RNN weisen beispielsweise GRU (Gated Recurrent Units) auf. Alternativ können die verdeckten Schichten des rekurrenten neuronalen Netzwerkes RNN auch jeweils mit LSTM-Modulen (Long Short Term Memory) aufgebaut werden. Ein LSTM-Modul bzw. eine LSTM-Zelle umfasst drei Torsorten, nämlich ein Eingangstor (input gate), ein Vergesstor (forget gate) und ein Ausgangstor (output gate). Die LSTM-Zelle ermöglicht auf diese Weise im Gegensatz zu anderen herkömmlichen rekurrenten neuronalen Netzen eine Art Erinnerung an frühere Erfahrungen, d.h. ein Kurzzeitgedächtnis, welches lange anhält. Bei dem GRU-Modul bzw. bei der GRU-Zelle kann das Vergesstor (forget gate) und das Eingangstor (input gate) zu einem einzigen Update Gate kombiniert sein.

Als Aktivierungsfunktion kann bei einer möglichen Ausführungsform eine Sigmoidfunktion verwendet werden, die eine Standardfunktion für eine binäre Klassenverteilung bildet.

Bei dem in Fig. 3 dargestellten Ausführungsbeispiel werden Sensordaten SD des überwachten Systems 2 verarbeitet. Diese können bei einer möglichen Ausführungsform aus einem Zwischenspeicher ausgelesen werden.

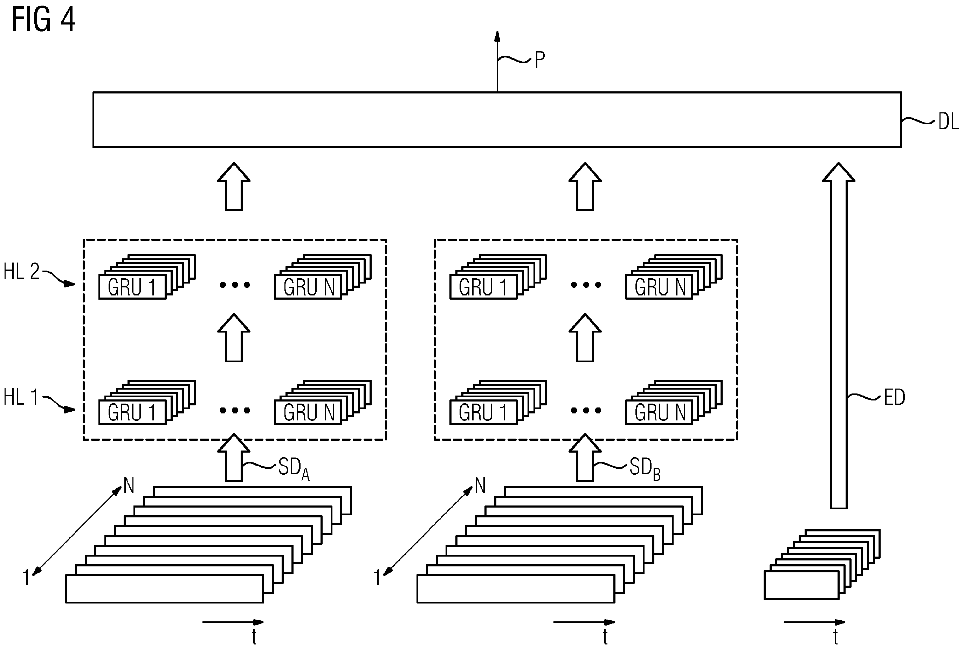

Fig. 4 zeigt ein weiteres Ausführungsbeispiel, bei dem zwei Arten verschiedener Input-Daten parallel verarbeitet werden. Bei dem in Fig. 4 dargestellten Ausführungsbeispiel werden die erfassten Zeitreihen der Sensordaten SD und/oder der Ereignisdaten ED zunächst zur Ermittlung von Zeitreihen gefiltert, die kurzfristige und langfristige zeitliche Trends innerhalb der erfassten Zeitreihen der Sensordaten und/oder der Ereignisdaten wiedergeben. Beispielsweise geben die Zeitreihendaten A die Sensorwerte der letzten x Scans an, um einen kurzfristigen Trend zu modellieren. Die in Fig. 4 dargestellten Zeitreihen bzw. Sensorwerte B geben einen längerfristigen Trend an. Wie in Fig. 4 dargestellt, werden die gefilterten Zeitreihen der Sensordaten A, B verdeckten Schichten HL verschiedener künstlicher neuronaler Netzwerke KNN zugeführt. Diese besitzen eine gemeinsame Ausgabeschicht DL zur Berechnung der Ausfallwahrscheinlichkeit der betreffenden überwachten Systemkomponente des medizinischen Systems 2. Bei dem in Fig. 4 dargestellten Ausführungsbeispiel werden zudem gefilterte Ereignisdaten ED direkt der Ausgabeschicht DL zugeführt.

Die durch die Ausgabeschicht DL (dense layer) berechnete Ausfallwahrscheinlichkeit P wird ständig einer Auswerteeinheit 7 zugeführt. Die Auswerteeinheit 7 wertet die Ausfallwahrscheinlichkeiten P auf Basis von vordefinierten Auswerteregeln R zur Bestimmung von Maßnahmen, welche einen teilweisen oder vollständigen Systemausfall des medizinischen Systems 2 verhindern, aus. In einer möglichen Ausführungsform wird durch die Auswerteeinheit 7 ein Systemausfallwarnsignal WS generiert, sobald die berechneten Ausfallwahrscheinlichkeiten P einer Systemkomponente mehrfach hintereinander ansteigen und zudem einen vorgegebenen Schwellenwert überschreiten. Alternativ kann ein Systemausfallwarnsignal WS bereits generiert werden, wenn die berechnete Ausfallwahrscheinlichkeit P einer Systemkomponente einen vorgegebenen Schwellenwert überschreitet. Der Schwellenwert ist hierbei für unterschiedliche Systemkomponenten unterschiedlich einstellbar. Die durch die Auswerteeinheit 7 angewendeten Regeln R können bei einer möglichen Ausführungsform aus einem Konfigurationsspeicher ausgelesen werden. In einer möglichen Ausführungsform sind die Regeln R anpassbar bzw. editierbar. In einer möglichen Ausführungsform können die Auswerteregeln R durch einen Nutzer des medizinischen Systems 2 oder einen Wartungstechniker konfiguriert werden. Das generierte Systemausfallwarnsignal WS kann an ein entferntes Service-Center übertragen werden. Ferner kann das generierte Systemausfallwarnsignal WS über eine Nutzerschnittstelle der Vorrichtung 1 und/oder des überwachten medizinischen Systems 2 ausgegeben werden.

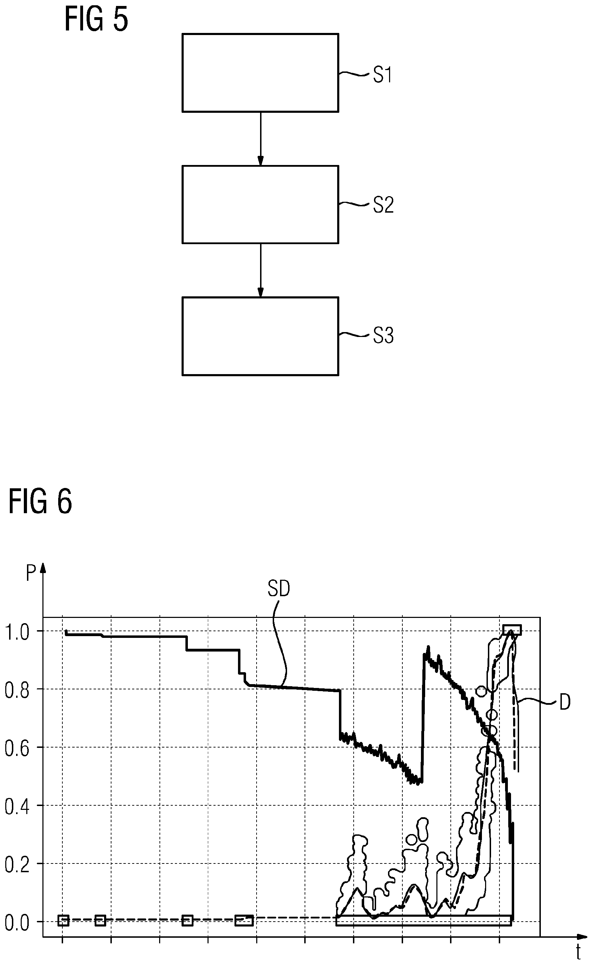

Fig. 6 illustriert beispielhaft die Erzeugung eines Systemausfallwarnsignals WS. Man erkennt die von der Ausgabeeinheit 6 ausgegebenen Systemausfallwahrscheinlichkeiten Pᵢ, ein Beispiel für einen Sensorwertverlauf SD und einen Durchschnittswertverlauf D. Sobald die berechneten Ausfallwahrscheinlichkeiten ansteigen und/oder einen Schwellenwert überschreiten, wird ein Systemausfallwarnsignal an ein Service-Center übertragen und dort weiter verarbeitet.

Fig. 5 zeigt ein Ablaufdiagramm zur Darstellung eines Ausführungsbeispiels eines erfindungsgemäßen Verfahrens zur Vorhersage von Systemausfällen bei einem medizinischen System.

Das Verfahren umfasst bei dem dargestellten Ausführungsbeispiel mehrere Schritte. In einem ersten Schritt S1 werden Sensordaten SD und/oder Ereignisdaten ED hinsichtlich mindestens einer Systemkomponente eines medizinischen Systems 2, beispielsweise einer Röntgenröhre, erfasst und zwischengespeichert.

In einem weiteren Schritt S2 werden die erfassten und zwischengespeicherten Sensordaten SD und/oder Ereignisdaten ED durch mindestens ein trainiertes künstliches neuronales Netzwerk KNN, insbesondere ein mehrschichtiges rekurrentes neuronales Netzwerk RNN, zur Berechnung einer Ausfallwahrscheinlichkeit P eines zukünftigen Ausfalls der betrachteten Systemkomponente des medizinischen Systems 2 ausgewertet.

Schließlich wird in Schritt S3 die berechnete Ausfallwahrscheinlichkeit P der überwachten Systemkomponente des medizinischen Systems ausgegeben. Beispielsweise wird die berechnete Ausfallwahrscheinlichkeit P an eine Auswerteeinheit 7 ausgegeben, welche die hintereinander erhaltenen Ausfallwahrscheinlichkeiten P betreffend einer Systemkomponente auf Basis von vordefinierten Auswerteregeln R zur Bestimmung von Maßnahmen, welche einen teilweisen oder vollständigen Systemausfall des medizinischen Systems 2 verhindern, auswertet. Für verschiedene Systemkomponenten können unterschiedliche Auswerteregeln R angewendet werden. Diese Auswerteregeln R werden beispielsweise aus einem Konfigurationsspeicher ausgelesen. Die in Schritt S3 berechnete Ausfallwahrscheinlichkeit P der Systemkomponente kann ferner über eine Nutzerschnittstelle einem Nutzer des medizinischen Gerätes oder einen Servicetechniker angezeigt werden. Bei einer möglichen Ausführungsform werden die berechneten Ausfallwahrscheinlichkeiten P einer Systemkomponente über die Zeit aufgezeichnet. Sobald die Ausfallwahrscheinlichkeiten P einer Systemkomponente mehrfach hintereinander ansteigen und/oder einen vorgegebenen Schwellenwert für diese Systemkomponente überschreiten, wird in einer möglichen Ausführungsform automatisch ein Systemausfallwarnsignal WS generiert, das beispielsweise an ein entferntes Service-Center übertragen werden kann oder über eine Nutzerschnittstelle des medizinischen Systems 2 ausgegeben wird.

Die in Schritt S1 erfassten Zeitreihen der Sensordaten SD und/oder Ereignisdaten ED können bei einer möglichen Ausführungsform zur Ermittlung von Zeitreihen gefiltert werden, die kurzfristige und langfristige zeitliche Trends innerhalb der erfassten Zeitreihen der Sensordaten und/oder Ereignisdaten wiedergeben. Diese gefilterten Zeitreihen der Sensordaten und/oder Ereignisdaten können verdeckten Schichten verschiedener künstlicher neuronaler Netze KNN zugeführt werden. Beispielsweise ist ein erstes künstliches neuronales Netzwerk für Zeitreihen vorgesehen, die einen kurzfristigen zeitlichen Trend wiedergeben. Ein anderes künstliches neuronales Netzwerk kann für Zeitreihen vorgesehen sein, das langfristige zeitliche Trends wiedergibt. Die verschiedenen verdeckten Schichten HL zur Auswertung der Zeitreihen hinsichtlich verschiedener zeitlicher Trends besitzen vorzugsweise eine gemeinsame Ausgabeschicht DL zur Berechnung der Ausfallwahrscheinlichkeit der betreffenden Systemkomponente. Bei der Systemkomponente handelt es sich beispielsweise um ein Filament eines Röntgensystems.

Sobald ein Systemausfallwarnsignal WS an ein Service-Center übertragen worden ist, können entsprechende Wartungsmaßnahmen bzw. Reparaturmaßnahmen eingeleitet werden, wobei eine Zeitreserve zur Verfügung steht, um die betroffene Systemkomponente rechtzeitig auszutauschen bzw. zu reparieren und so einen Systemausfall der Systemkomponente und somit des gesamten medizinischen Systems zuverlässig zu verhindern. Das erfindungsgemäße Verfahren ist in der Lage komplexe Muster zu erkennen, die auf einen Systemausfall einer Systemkomponente hindeuten. Mithilfe des erfindungsgemäßen Verfahrens ist es möglich einen Systemausfall zuverlässig und präzise vorherzusagen. Durch das Zusammenführen verschiedener Input-Datentypen, insbesondere Ereignisdaten und Sensordaten, ist es ferner möglich falsch positive Vorhersagen zu reduzieren.

## Patentansprüche

1. Verfahren zur Vorhersage von Systemausfällen bei einem medizinischen System (2) mit den Schritten:
(a) Erfassen (S1) von Sensordaten (SD) und/oder Ereignisdaten (ED) hinsichtlich mindestens einer Systemkomponente des medizinischen Systems (2);
(b) Auswerten (S2) der erfassten Sensordaten (SD) und/oder der erfassten Ereignisdaten (ED) durch mindestens ein trainiertes künstliches neuronales Netzwerk (5) zur Berechnung einer Ausfallwahrscheinlichkeit (P) eines zukünftigen Ausfalls der Systemkomponente des medizinischen Systems (2); und
(c) Ausgeben (S3) der berechneten Ausfallwahrscheinlichkeit (P) der Systemkomponente des medizinischen Systems.

2. Verfahren nach Anspruch 1,
wobei das künstliche neuronale Netzwerk (5) ein mehrschichtiges rekurrentes neuronales Netzwerk (RNN) aufweist.

3. Verfahren nach Anspruch 1 oder 2,
wobei berechnete Ausfallwahrscheinlichkeiten (P) an eine Auswerteeinheit (7) ausgegeben werden, welche die erhaltenen Ausfallwahrscheinlichkeiten (P) auf Basis von vordefinierten Auswerteregeln (R) zur Bestimmung von Maßnahmen, welche einen teilweisen oder vollständigen Systemausfall des medizinischen Systems (2) verhindern, auswertet.

4. Verfahren nach Anspruch 3,
wobei die Auswerteeinheit (7) ein Systemausfallwarnsignal (WS) generiert, sobald die berechneten Ausfallwahrscheinlichkeiten (P) mehrfach hintereinander ansteigen und/oder einen vorgegebenen Schwellenwert überschreiten.

5. Verfahren nach Anspruch 4,
wobei das generierte Systemausfallwarnsignal (WS) an ein entferntes Service-Center übertragen wird und/oder über eine Nutzerschnittstelle ausgegeben wird.

6. Verfahren nach einem der vorangehenden Ansprüche 1 bis 5,
wobei erfasste Zeitreihen der Sensordaten (SD) und/oder der Ereignisdaten (ED) zur Ermittlung von Zeitreihen gefiltert werden, die kurzfristige und langfristige zeitliche Trends innerhalb der erfassten Zeitreihen der Sensordaten (SD) und/oder der Ereignisdaten (ED) wiedergeben.

7. Verfahren nach Anspruch 6,
wobei die gefilterten Zeitreihen der Sensordaten (SD) und/oder der Ereignisdaten (ED) verdeckten Schichten verschiedener künstlicher neuronaler Netzwerke (5) zugeführt werden, die eine gemeinsame Ausgabeschicht zum Berechnen der Ausfallwahrscheinlichkeit (P) der Systemkomponente des medizinischen Systems (2) besitzen.

8. Verfahren nach einem der vorangehenden Ansprüche 1 bis 7,
wobei die Systemkomponente durch ein Filament eines Röntgensystems gebildet wird.

9. Vorrichtung zur Vorhersage von Systemausfällen bei einem medizinischen System (2), wobei die Vorrichtung (1) aufweist:
- eine Erfassungseinheit (3) zur Erfassung von Sensordaten (SD) und/oder Ereignisdaten (ED) hinsichtlich mindestens einer Systemkomponente des medizinischen Systems (2);
- eine Berechnungseinheit (4) mit mindestens einem darin implementierten künstlichen neuronalen Netzwerk (5) zur Berechnung einer Ausfallwahrscheinlichkeit (P) für einen zukünftigen Ausfall der Systemkomponente des medizinischen Systems (2), und
- eine Ausgabeeinheit (6) zur Ausgabe der berechneten Ausfallwahrscheinlichkeit (P) der Systemkomponente des medizinischen Systems (2).

10. Vorrichtung nach Anspruch 9,
wobei das künstliche neuronale Netzwerk (5) ein mehrschichtiges rekurrentes neuronales Netzwerk aufweist, das mehrere verdeckte Schichten (HL) und eine Ausgabeschicht (DL) enthält.

11. Vorrichtung nach Anspruch 10,
wobei die verdeckten Schichten (HL) des rekurrenten neuronalen Netzwerkes (5) jeweils LSTM-Module oder GRU-Module aufweisen.

12. Vorrichtung nach Anspruch 10 oder 11,
wobei eine erste verdeckte Schicht HL₁) des in der Berechnungseinheit (4) implementiertes rekurrenten neuronalen Netzwerkes (5) eine Eingabeschicht bildet, die über eine Schnittstelle Sensordaten (SD) und/oder Ereignisdaten (ED) von einem Zwischenspeicher der Erfassungseinheit (3) erhält.

13. Vorrichtung nach einem der vorangehenden Ansprüche 9 bis 12,
wobei Filtereinheiten vorgesehen sind, welche Zeitreihen der durch die Erfassungseinheit (3) erfassten Sensordaten (SD) und/oder Ereignisdaten (ED) zur Ermittlung gefilterter Zeitreihen filtern, die kurzfristige und langfristige zeitliche Trends innerhalb der erfassten Sensordaten (SD) und/oder Ereignisdaten (ED) wiedergeben,
wobei die durch die Filtereinheiten gefilterten Zeitreihen Eingabeschichten verschiedener innerhalb der Berechnungseinheit implementierter rekurrenter neuronaler Netzwerke (5) zugeführt werden.

14. Vorrichtung nach einem der vorangehenden Ansprüche 9 bis 13,
wobei die Ausgabeeinheit (6) die durch die Berechnungseinheit (4) berechnete Ausfallwahrscheinlichkeiten (P) der Systemkomponente an eine Auswerteeinheit (7) ausgibt, welche die Ausfallwahrscheinlichkeiten (P) auf Basis vordefinierter Auswerteregeln (R) zur Generierung eines Systemausfallwarnsignales (WS) auswertet, das einen teilweisen oder vollständigen Systemausfall des medizinischen Systems (2) anzeigt.

15. Vorrichtung nach Anspruch 14,
wobei die Auswerteeinheit (7) eine Datenschnittstelle zur Ausgabe des generierten Systemausfallwarnsignales (WS) an ein Service-Center und/oder eine Nutzerschnittstelle zur Ausgabe des generierten Systemausfallwarnsignales (WS) an einen Nutzer des medizinischen Systems (2) aufweist.
